**Europäisches Patentamt**

**European Patent Office**

**Office européen des brevets**

(19)

(11) Veröffentlichungsnummer: **0 024 345**
**B1**

(12) **EUROPÄISCHE PATENTSCHRIFT**

(45) Veröffentlichungstag der Patentschrift:
27.07.83

(51) Int. Cl.³: **C 12 N 9/18** // C12Q1/60, C12R1/38

(21) Anmeldenummer: **80104796.0**

(22) Anmeldetag: **13.08.80**

(54) Verfahren zur Gewinnung von Cholesterinesterase.

(30) Priorität: **20.08.79 DE 2933646**

(43) Veröffentlichungstag der Anmeldung:
**04.03.81 Patentblatt 81/9**

(45) Bekanntmachung des Hinweises auf die Patenterteilung:
**27.07.83 Patentblatt 83/30**

(84) Benannte Vertragsstaaten:
**AT BE CH DE FR GB IT LI LU NL SE**

(56) Entgegenhaltungen:
**DE-A-2 527 068**
**AGR. BIOL. CHEM., Band 40, Nr. 8, 1976, T. UWAJIMA et al.: »Production of cholesterol esterase by pseudomonas fluorescens«, Seiten 1605—1609**
**CHEMICAL ABSTRACTS, Band 79, Nr. 25, 24. Dezember 1973, Seite 205, Zusammenfassung Nr. 144876u, Columbus, OHIO, US**
**CHEMICAL ABSTRACTS, Band 89, 3. Juli 1978, Seite 415, Zusammenfassung Nr. 4587g, Columbus, Ohio, US**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Beaucamp, Klaus, Dr., Von-Kühlmann-Strasse 15, D-8132 Tutzing (DE)**
Erfinder: **Nelboeck, Michael, Dr., Bareislweg 21, D-8132 Tutzing (DE)**
Erfinder: **Gauhl, Helmgard, Kustermannstrasse 31, D-8132 Tutzing (DE)**
Erfinder: **Seidel, Hans, Dr., Waxensteinstrasse 6, D-8132 Tutzing (DE)**
Erfinder: **Gruber, Wolfgang, Dr., Am Oberanger 7, D-8132 Tutzing-Unterzeismering (DE)**
Erfinder: **Brunner, Herwig, Dr., Paradeisstrasse 20 D, D-8120 Weilheim (DE)**

(74) Vertreter: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)**

**0 024 345**

## Verfahren zur Gewinnung von Cholesterinesterase

Die Erfindung betrifft ein Verfahren zur Gewinnung von Cholesterinesterase aus Mikroorganismen. Cholesterinesterase spielt eine wichtige Rolle in der klinischen und biochemischen Analytik, seit Verfahren zur enzymatischen Bestimmung von Cholesterin entwickelt wurden. Da ein großer Teil des Cholesterins in biologischem Material in Form von Estern vorliegt, ermöglicht die gemeinsame Verwendung von Cholesterinesterase und Cholesterin oxidierenden Enzymen, wie Cholesterinoxidase oder Cholesterindehydrogenase, eine vollenzymatische Bestimmung auch von Cholesterinestern. Dies ist bekannt aus DE-PS 2 264 847. Als besonders geeignet hat sich im Rahmen der Cholesterinesterase-Bestimmung dabei das Enzym aus Mikroorganismen erwiesen (DE-OS 2 506 712.3). Ein Nachteil der bisher aufgefundenen Mikroorganismen mit einem die Aufarbeitung lohnenden Gehalt an Cholesterinesterase besteht jedoch in den relativ geringen Ausbeuten an Enzymaktivität, die dabei erhalten werden.

Normalerweise erfolgt bei den bekannten Verfahren die Züchtung in einem Nährmedium, welches einen Induktor enthält. Unter »Induktor« wird hierbei eine Substanz verstanden, welche den Mikroorganismus dazu anregt, das gewünschte Enzym überhaupt oder in größerer Menge zu bilden als ohne Induktor. Denn normalerweise benötigten Mikroorganismen keine Cholesterinesterase, da ihnen genügend andere Nahrungsquellen zur Verfügung stehen und die Bildung eines nicht benötigten Enzyms für die Zelle unwirtschaftlich ist. Induktoren bestehen daher aus Cholesterinestern oder chemisch ähnlichen Verbindungen.

Nunmehr wurde überraschenderweise gefunden, daß bei Verwendung eines bestimmten, den Cholesterinestern chemisch fernstehenden Induktors, um ein Vielfaches höhere Aktivitäten erzielt werden können, als dies bisher möglich war. Das erfindungsgemäße Verfahren zur Gewinnung von Cholesterinesterase aus Mikroorganismen ist daher dadurch gekennzeichnet, daß man einen zur Cholesterinesterase-Bildung befähigten Mikroorganismus in einem geeigneten Nährmedium in Gegenwart von Lecithin als Induktor züchtet und das Enzym aus der Kulturflüssigkeit oder/und den Zellen gewinnt.

Vorzugsweise wird der erfindungsgemäß eingesetzte Induktor auch als Kohlenstoffquelle, insbesondere als alleinige Kohlenstoffquelle, verwendet. Es ist jedoch auch möglich, gesonderte Kohlenstoffquellen zuzusetzen, beispielsweise Maisquellwasser, Peptone, Hefeextrakte sowie, weniger geeignet, Zucker oder Polyalkohole, wie Glycerin. Als besonders geeignet unter den verschiedenen Lecithinen erwies sich Sojalecithin, aber auch andere Lecithinarten, wie Eilecithin oder Hirnlecithin, ergaben sehr gute Resultate.

Die eingesetzte Lecithinmenge liegt im allgemeinen zwischen etwa 0,1 und 5 Gew.-%, bezogen auf das Volumen des Nährmediums. Bei Verwendung von Lecithin als Induktor und alleinige Kohlenstoffquelle wurden besonders gute Ergebnisse bei einer Menge von 0,5 bis 2 Gew.-% erhalten.

Für die Erfindung eignen sich im Prinzip alle Mikroorganismen, welche Cholesterinesterase in einer die Aufarbeitung lohnenden Menge zu bilden vermögen. Derartige Mikroorganismen sind in großer Zahl bekannt. So sind beispielsweise geeignet:

| | |
|---|---|
| Candida rugosa | ATCC 14830 |
| Rhizopus spec. | DSM 695 |
| Aspergillus spec. | DSM 698 |
| Streptomyces aureoverticillium | ESM 40080 |
| Streptomyces cyaneofuscatus | DSM 40148 |
| Streptomyces griseomycini | DSM 40159 |
| Streptomyces longisporus-fl. | DSM 40165 |
| Streptomyces malachiticus | DSM 40167 |
| Streptomyces reseolus | DSM 40174 |
| Streptomyces toxytricini | DSM 40178 |
| Streptomyces variabilis | DSM 40179 |
| Streptomyces spec. | DSM 687 |
| Streptomyces autotrophicus | DSM 40011 |
| Streptomyces canescens | DSM 40528 |
| Streptomyces chartreusis | DSM 40085 |
| Streptomyces michiganensis | DSM 40015 |
| Streptomyces murinus | DSM 40091 |
| Streptomyces hachijoensis | DSM 40114 |
| Streptomyces caelestes | DSM 40084 |
| Streptomyces tendae | DSM 40101 |
| Nocardia rubra | DSM 43008 |
| Candida mycoderma | DSM 688 |
| Candida albicans | DSM 689 |
| Candida albicans | DSM 690 |

2

| Candida albicans | DSM 691 |
| Candida spec. | DSM 692 |
| Cunninghamella elegans | DSM 693 |
| Mucor mucedo | DSM 694 |
| Penicillium spec. | DSM 696 |
| Aspergillus spec. | DSM 697 |
| Pseudomonas fluorescens | ATCC 31156 und |
| Pseudomonas fluorescens | ATCC 948. |

Besonders bevorzugt werden Pseudomonas spec. DSM 1280 und 1281.

Ein besonders bevorzugtes Nährmedium, welches besonders für die Pseudomonas-Arten geeignet ist, enthält darüber hinaus noch üblicherweise zugesetzte Salze und Spurenelemente und sollte durch Zugabe eines geeigneten Puffers auf einen pH-Wert zwischen etwa 5 und 9, vorzugsweise 6 und 8, eingestellt werden. Als Puffer wird Phosphatpuffer bevorzugt. Darüber hinaus enthält das Nährmedium zweckmäßig noch Ammonium-, Chlor-, Fe-, Cu-, Zn-, Mg- und Ca-Ionen, abgesehen von den Alkaliionen des Phosphatpuffers. Phosphat liegt dabei zweckmäßig in einer Konzentration zwischen 0,4 und 2 Gew.-% vor, jedoch können auch größere oder kleinere Konzentrationen eingesetzt werden.

Ein im Rahmen der Erfindung besonders bevorzugtes Nährmedium besitzt etwa folgende Zusammensetzung, jeweils bezogen auf 1 l Flüssigkeit:

5 bis 10 g, vorzugsweise 6 bis 8 g, $Na_2HPO_4 \cdot 2 H_2O$;
1 bis 5 g, vorzugsweise 2 bis 4 g, $KH_2PO_4$;
0,2 bis 2, vorzugsweise 0,8 bis 1,2 g $NH_4Cl$;
0,01 bis 0,1, vorzugsweise 0,3 bis 0,7 g, NaCl;
0,01 bis 1 ml 1%ige $FeCl_3$-Lösung;
0,01 bis 1 ml 0,2%ige $CuCl_2$-Lösung;
0,01 bis 1 ml 1%ige Zinksulfat-Lösung;
0,1 bis 10 ml 10%ige $CaCl_2$-Lösung;
1 bis 20, vorzugsweise 3 bis 10 ml 12%ige $MgSO_4$-Lösung;
0,1 bis 5, vorzugsweise 0,5 bis 2 Gew.-% Sojalecithin.

Die Züchtung der besonders bevorzugten Mikroorganismen in den obigen Nährmedien wird unter aeroben Bedingungen durchgeführt. Es eignen sich sowohl Schüttelkultur als auch belüftete Submerskultur. Die Temperatur kann zwischen etwa 15 und etwa 45° C liegen, bevorzugt werden 25 bis 35° C. Maximale Enzymausbeuten erhält man im allgemeinen bereits nach 1- bis 2tägiger Kulturdauer.

Die Cholesterinesterase kann sowohl im Kulturmedium als auch in den Zellen auftreten. Durch Zusatz von oberflächenaktiven Mitteln, insbesondere von nichtionogenen Mitteln, die vorzugsweise dem Typus der Polyoxyäthylenester und -äther mit Alkyl- und Aralkylresten angehören, läßt sich bei vielen Mikroorganismen das Verteilungsmuster zwischen Kulturbrühe und Zelle beeinflussen, gewöhnlich im Sinne einer Erhöhung der extrazellulären Aktivität auf Kosten der intrazellulären Aktivität; bei ionogenen oberflächenaktiven Mitteln verläuft die Änderung der Verteilung jedoch gelegentlich in umgekehrter Richtung.

Nach beendeter Züchtung wird die Cholesterinesterase aus der Zellmasse und/oder dem Kulturfiltrat nach üblichen Methoden isoliert und gegebenenfalls gereinigt. Für viele Zwecke eignet sich jedoch bereits ein ungereinigtes Rohprodukt, welches im wesentlichen nur aus aufgeschlossener Zellmasse besteht. Zum Aufschließen eignen sich die dem Fachmann hierfür bekannten Methoden, die hier keiner näheren Erläuterung bedürfen. Sowohl aus dem Kulturfiltrat als auch aus der aufgeschlossenen Zellmasse läßt sich nach Abtrennung von unlöslichen Bestandteilen das Enzym durch Fällung mit üblichen Fällungsmitteln, beispielsweise Salzen, wie Ammoniumsulfat, oder organischen Lösungsmitteln, wie Aceton oder Alkohol, fällen und dann unter Anwendung der üblichen Fraktionierungsmethoden, wie Chromatographie und Fällung, weiter aufreinigen.

Die folgenden Beispiele erläutern die Erfindung weiter.

Beispiel 1

Pseudomonas spec. DSM 1280, aus der Tiefkühlampulle auf Schrägröhrchen gebracht, wird im Hauptkulturmedium zwei Tage bei 30° C aerob (Schüttelkolben) vorkultiviert und dann zu 10% in ein Medium überimpft, welches pro Liter folgende Zusammensetzung aufweist:

7 g $Na_2HPO_4 \cdot 2 H_2O$
3 g $KH_2PO_4$
1 g $NH_4Cl$
0,05 g NaCl

3

0,1 ml FeCl$_3$, 1%
0,1 ml CuCl$_2$, 0,2%
0,1 ml ZnSO$_4$, 1%
1,0 ml CaCl$_2$, 10%
5,0 ml MgSO$_4$, 12%
1,5% Soja-Lecithin, pH 7,0.

Die Kultivierung erfolgt bei 30°C aerob im Schüttelkolben. Nach 1 bis 3 Tagen werden Aktivitäten von etwa 15 000 U/l (Überstand und Biomasse; Substrat: Cholesteryl-oleat) erhalten.

Etwa gleiche Ausbeuten werden erhalten, wennn unter gleichen Bedingungen anstelle von Pseudomonas spec. DSM 1280 Pseudomonas spec. DSM 1281 verwendet wird.


Beispiel 2

Aus einer nach Beispiel 1 erhaltenen Kulturlösung wurden die unlösliche Zellmasse abzentrifugiert und zur Cholesterinester-Bestimmung eingesetzt. Die Bestimmung erfolgte gemäß folgenden Reaktionsgleichungen:

1)  Cholesterinester + H$_2$O $\xrightarrow{\text{Chol-Esterase}}$ Cholesterin + Fettsäure

2)  Cholesterin + 1/2 O$_2$ $\xrightarrow{\text{Chol-OD/Katalase}}$ Cholestenon + H$_2$O$_2$

(Messung der Cholestenonbildung bei 240 nm)


Zur Messung wurden folgende Lösungen verwendet:

1)  Phosphatpuffer 0,5 M, pH 7,5, 0,4% Thesit
2)  Cholesterinoleat c = 4 in Thestidioxan (v/v = 1/1)
3)  H$_2$O$_2$ ca. 0,6 M (5 ml Perhydrol/100 ml)
4)  Katalase (0,01 mg Protein/ml)
5)  Cholesterinoxidase (mind. 50 U/ml)
6)  Kulturlösung (bei ca. 5000 U/l 1 : 5 mit H$_2$O verdünnt, 0,01 ml → Test).

Zur Messung wurden 2,95 ml Lösung 1) mit 0,02 ml Lösung 3) gemischt. Nach 5 Minuten wurden 0,01 ml Lösung 6) und 0,02 ml Lösung 5) zugesetzt und nach 1 Minute die Reaktion durch Zugabe von 0,1 ml Lösung 2) gestartet.

Die Berechnung wird wie folgt durchgeführt:

$$\frac{3,12 \cdot 5 \cdot 1000}{15,5 \cdot 0,01} \cdot \Delta \text{ E/min} = \text{U/l Kulturlösung.}$$


Beispiel 3

Candida rugosa ATCC 14830, aus einer Tiefkühlampulle auf Schrägröhrchen gebracht, wird in ein Kulturmedium der nachstehenden Zusammensetzung überimpft und 48 Stunden bei 28 bis 30°C aerob (Schüttelkolben 20/100) kultiviert. Dann wird mit 10% Inokulum in ein Medium überimpft, welches folgende Zusammensetzung aufweist (Angaben pro Liter):

20 g Sojamehl GeFu 988 SUP
20 g Stärke, löslich
5 g K$_2$HPO$_4$ · 3 H$_2$O
1 g MgSO$_4$ · 7 H$_2$O
1 g (NH$_4$)$_2$SO$_4$
1,5% Sojalecithin
pH 6,6 bis 6,8.

Die Kultivierung erfolgt bei ca. 28°C aerob. Nach 3 bis 4 Tagen werden Aktivitäten von 1500 bis 2000 U/l (Überstand; Testsubstrat: Cholesterinoleat) erhalten.

**Patentansprüche**

1. Verfahren zur Gewinnung von Cholesterinesterase aus Mikroorganismen, dadurch gekennzeichnet, daß man einen zur Cholesterinesterase-Bildung befähigten Mikroorganismus in einem geeigneten Nährmedium in Gegenwart von Lecithin als Induktor züchtet und das Enzym aus der Kulturflüssigkeit oder/und den Zellen gewinnt.

2. Verfahren nach Anspruch 1, dadurch gekennzeichnet, daß man Sojalecithin zusetzt.

3. Verfahren nach Anspruch 1 oder 2, dadurch gekennzeichnet, daß man in Gegenwart von 0,5 bis 2 Gew.-% Lecithin züchtet.

4. Verfahren nach einem der vorhergehenden Ansprüche, dadurch gekennzeichnet, daß man dem Medium 0,4 bis 2 Gew.-% Phosphat zusetzt.

5. Verfahren nach einem der vorhergehenden Ansprüche, dadurch gekennzeichnet, daß man als Mikroorganismus Pseudomonas spec. DSM 1280 oder DSM 1281 verwendet.

6. Verfahren nach einem der vorhergehenden Ansprüche, dadurch gekennzeichnet, daß Lecithin auch als Kohlenstoffquelle verwendet wird.

7. Verfahren nach einem der vorhergehenden Ansprüche, dadurch gekennzeichnet, daß man ein durch Zugabe eines geeigneten Puffers auf einen pH-Wert zwischen 5 und 9 eingestelltes, Salze und Spurenelemente enthaltendes Nährmedium verwendet.

8. Verfahren nach Anspruch 7, dadurch gekennzeichnet, daß man Phosphatpuffer verwendet.

9. Verfahren nach Anspruch 8, dadurch gekennzeichnet, daß man ein Nährmedium verwendet, welches bezogen auf 1 Liter Flüssigkeit

5 bis 10 g $Na_2HPO_4 \times 2\ H_2O$,
1 bis 5 g $KH_2PO_4$,
0,2 bis 2 g $NH_4Cl$,
0,01 bis 0,1 g $NaCl$,
0,01 bis 1 ml 1%ige $FeCl_3$-Lösung,
0,01 bis 1 ml 0,2%ige $CuCl_2$-Lösung,
0,01 bis 1 ml 1%ige Zinksulfat-Lösung,
0,1 bis 10 ml 10%ige $CaCl_2$-Lösung,
1 bis 20 ml 12%ige $MgSO_4$-Lösung und
0,1 bis 5 Gew.-% Sojalecithin enthält.

10. Verfahren nach einem der vorhergehenden Ansprüche, dadurch gekennzeichnet, daß die Züchtung bei 25 bis 35° C durchgeführt wird.

**Claims**

1. Process for the obtaining of cholesterol esterase from micro-organisms, characterised in that one cultures a micro-organism capable of cholesterol esterase formation in a suitable nutrient medium in the presence of lecithin as inductor and obtains the enzyme form the culture liquid and/or the cells.

2. Process according to claim 1, characterised in that one adds soya lecithin.

3. Process according to claim 1 or 2, characterised in that one cultures in the presence of 0.5 to 2 wt.% lecithin.

4. Process according to one of the preceding claims, characterised in that one adds 0.4 to 2 wt.% phosphate to the medium.

5. Process according to one of the preceding claims, characterised in that as micro-organism one uses Pseudomonas spec. DSM 1280 or DSM 1281.

6. Process according to one of the preceding claims, characterised in that lecithin is also used as carbon source.

7. Process according to one of the preceding claims, characterised in that one uses a nutrient medium containing salts and trace elements adjusted to a pH value between 5 and 9 by the addition of a suitable buffer.

8. Process according to claim 7, characterised in that one uses phosphate buffer.

9. Process according to claim 8, characterised in that one uses a nutrient medium which, referred to 1 litre of liquid, contains:

5 to 10 g. $Na_2HPO_4 \times 2\ H_2O$,
1 to 5 g. $KH_2PO_4$,
0.2 to 2 g. $NH_4Cl$,
0.01 to 0.1 g. $NaCl$,
0.01 to 1 ml. 1% $FeCl_3$ solution,
0.01 to 1 ml. 0.2% $CuCl_2$ solution,
0.01 to 1 ml. 1% zinc sulphate solution,

**0 024 345**

0.1 to 10 ml. 10% $CaCl_2$ solution,
1 to 20 ml. 12% $MgSO_4$ solution and
0.1 to 5 wt.% soya lecithin.

10. Process according to one of the preceding claims, characterised in that the culturing is carried out at 25 to 35° C.


### Revendications

1. Procédé de préparation de la cholestérol estérase à partir de microorganismes, caractérisé en ce qu'on cultive un microorganisme capable de former de la cholestérol estérase, dans un milieu nutritif approprié, en présence de lécithine comme inducteur, et en ce qu'on recueille l'enzyme à partir du liquide de culture ou/et des cellules.

2. Procédé suivant la revendication 1, caractérisé en ce qu'on ajoute de la lécithine de soja.

3. Procédé suivant la revendication 1 ou 2, caractérisé en ce qu'on cultive en présence de 0,5 à 2% en poids de lécithine.

4. Procédé suivant l'une des revendications précédentes, caractérisé en ce qu'on ajoute au milieu 0,4 à 2% en poids de phosphate.

5. Procédé suivant l'une des revendications précédentes, caractérisé en ce qu'on utilise comme microorganisme Pseudomonas spec. DSM 1280 ou DSM 1281.

6. Procédé suivant l'une des revendications précédentes, caractérisé en ce que la lécithine est également utilisé comme source de carbone.

7. Procédé suivant l'une des revendications précédentes, caractérisé en ce qu'on utilise un milieu nutritif ajusté à une valeur de pH entre 5 et 9 par addition d'un tampon approprié, contenant des sels et des oligo-éléments.

8. Procédé suivant la revendication 7, caractérisé en ce qu'on utilise un tampon phosphate.

9. Procédé suivant la revendication 8, caractérisé en ce qu'on utilise un milieu nutritif qui contient, pour 1 litre de liquide:

5 à 10 g de $Na_2HPO_4 \cdot 2 H_2O$;
1 à 5 g de $KH_2PO_4$;
0,2 à 2 g de $NH_4Cl$;
0,01 à 0,1 g de NaCl;
0,01 à 1 ml de solution de $FeCl_3$ à 1%;
0,01 à 1 ml de solution de $CuCl_2$ à 0,2%;
0,01 à 1 ml de solution de sulfate de zinc à 1%;
0,1 à 10 ml de solution de $CaCl_2$ à 10%;
1 à 20 ml de solution de $MgSO_4$ à 12%; et
0,1 à 5% en poids de lécithine de soja.

10. Procédé suivant l'une des revendications précédentes, caractérisé en ce que la culture est effectuée à 25 à 35° C.

6